# EUROPEAN PATENT SPECIFICATION

(11) **EP 4 627 534 B1**
(45) Date of publication and mention of the grant of the patent: **14.01.2026**
(21) Application number: 23812868.0
(22) Date of filing: 21.11.2023
(51) Int. Cl.: G06T 11/00

(54) **ENSURING QUALITY FOR EVENTS OF VARYING CONTRAST AGENT IN 3D DUAL-ENERGY X-RAY IMAGING**
QUALITÄTSSICHERUNG FÜR EREIGNISSE MIT VARIIERENDEM KONTRASTMITTEL IN DER 3D-DOPPELENERGIE-RÖNTGENBILDGEBUNG
GARANTIE DE QUALITÉ POUR DES ÉVÉNEMENTS DE VARIATION D'AGENT DE CONTRASTE DANS UNE IMAGERIE PAR RAYONS X À DOUBLE ÉNERGIE 3D

(30) Priority: 01.12.2022 EP 22210824
(43) Date of publication of application: 08.10.2025
(73) Proprietor: Koninklijke Philips N.V., 5656 AG Eindhoven (NL)
(72) Inventor: SCHÄFER, Dirk, 5656 AG Eindhoven (NL); BRENDEL, Bernhard, Johannes, 5656AG Eindhoven (NL)
(74) Representative: Philips Intellectual Property & Standards
(86) International application number: PCT/EP2023/082482
(87) International publication number: WO 2024/115188

(56) References cited:
- US-A1- 2020 281 543
- ROELANDTS TOM ET AL: "The reconstructed residual error: A novel segmentation evaluation measure for reconstructed images in tomography", COMPUTER VISION AND IMAGE UNDERSTANDING, ACADEMIC PRESS, US, vol. 126, 4 June 2014 (2014-06-04), pages 28 - 37, XP029036769, ISSN: 1077-3142, DOI: 10.1016/J.CVIU.2014.05.007

## Description

### FIELD OF THE INVENTION

The invention relates to a system for medical image processing, to an imaging arrangement, to a related method, to a computer program element and to a computer readable medium.

### BACKGROUND OF THE INVENTION

Coronary CTA (computed tomography angiography) imaging is gaining importance as a non-invasive first line diagnostic tool for suspected coronary artery disease. Specifically, CT perfusion or CT derived IMR assessment garnered recent interest since these approaches may support the diagnosis of microvascular dysfunction and thus address a long-standing problem.

Such imaging setup use contrast agent to boost contrast of structures that are natively next to transparent to X-radiation. Dual layer detectors for C-arm systems may also be used in the near future to achieve interventional spectral imaging.

Projection data acquired with a dual layer detector (or any other technique that allows acquiring energy resolved projection data from the same viewing angle) enables material decomposition on the projections. If Iodine or any other contrast agent (CA) is administered before or during acquisition, one possible result of material decomposition is a 2D CA map that indicates how much CA is in the X-ray beam for each pixel of the projection. If projections are recorded along a 3D acquisition trajectory, it is possible to reconstruct a 3D CA map from the entirety of collected 2D contrast agent maps. The 3D CA map represents a spatial distribution of the quantitative CA concentrations of the scanned object. Once a correct 3D CA map is available, a virtual non-contrast (VNC) image can be derived.

Most accurate 3D CA maps and VNC (virtual-non-contrast) images, or VCs (virtual contrast only) images may be obtained, if all parts of the scanned object that contribute to the 2D CA maps are located inside the reconstructable FOV (field of view). A potential application of quantitative 3D CA maps / VNC images is the discrimination between a fresh bleeding and staining iodine, which both appear as hyperdense on a post-treatment CBCT stroke scan. This may be useful also in other types of interventional CBCT imaging, e.g. multi-phase liver tumor assessment and ablation, general oncology or urology and others.

Alas, it has been found that contrasted spectral imagery reconstructions can be inflicted with artifacts that can potentially occlude relevant medical details and undermine usefulness of the imagery. As for related art, reference is made to ROELANDTS TOM ET AL: "The reconstructed residual error: A novel segmentation evaluation measure for reconstructed images in tomography",COMPUTER VISION AND IMAGE UNDERSTANDING, ACADEMIC PRESS, US,vol. 126, 4 June 2014, pages 28-37, ISSN: 1077-3142, DOI: 10.1016/J.CVIU.2014.05.007 who describe comparing forward projected segmentations with the original projection data for quality purposes. US 2020/281543 A1 discloses multi-energy spectral CT reconstructions, including the segmentation of regions in which contrast agent is found.

### SUMMARY OF THE INVENTION

There may therefore be a need for improved contrast agent assisted tomographic imaging.

The invention is defined by the appended claims. Accordingly, an object of the present invention is achieved by the subject matter of the independent claims where further embodiments are incorporated in the dependent claims. It should be noted that the following described aspect of the invention equally applies to the related method, to the imaging arrangement, to the computer program element and to the computer readable medium.

According to a first aspect of the invention there is provided a system for image processing, comprising:
an input interface for receiving, when the system is in use, a volume image reconstructed from spectrally processed projection data acquired by a spectral imaging apparatus (IA) whilst contrast agent is present in a field of view of the spectral imaging apparatus during acquisition;
a segmentor component configured to segment the volume image for contrast agent contributions to obtain a segmented volume image;
a forward-projector configured to forward-project the segmented volume image onto the spectrally processed projection data;
a quality checker configured to establish quality indictor data indicative of a change of a concentration of the contrast agent in the field of view during the acquisition, based on a mismatch between the forward-projected segmented volume image and the spectrally processed projection data, and
an output interface for providing output data including the quality indicator data.

The spectrally processed projection data may include for example VC projection imagery, obtained by using any one of a range of spectral image processing techniques, such as a material decomposition algorithm of any suitable kind.

The output data may be used by a user in assessing a quality of the reconstructed volume image, in particular, to what extent the reconstructed volume image is corrupted by image artifacts caused by contrast agent's concentration fluctuating in the imager's FOV during the projection data acquisition.

In embodiments, the system comprises a graphics display generator or visualizer, configured to generate, based on the output data, a graphics display for display on a display device, wherein in the graphics display the mismatch, if any, is localized in at least part of the projection data or in the volume image.

In embodiments, the system comprises a control interface configured to request, based on the output data, any one or more of: i) issuing an alert signal, ii) additional processing in relation to the projection data, iii) an additional acquisition of new projection data.

In embodiments, the alert signal is indicative whether such additional processing or additional acquisition is warranted, or that such additional processing or additional acquisition is recommended, and wherein the additional processing or additional acquisition is requested based on user input.

In embodiments, the alert signal includes any one or more of: i) an audio alert signal, ii) a haptic feedback-signal, iii) a visual alert signal.

In embodiments, the additional processing includes any one or more of i) an additional reconstruction operation by a reconstructor to reconstruct a second volume image based on the new projection data or based on the forward projections obtained by forward projector, ii) a correcting by a corrector of the volume image for the mismatch, based on the said output data.

In another aspect there is provided an imaging arrangement including the system, and further including one or more of: i) the display device, ii) the imaging apparatus.

In another aspect there is provided a method of image processing, comprising:-
receiving a volume image reconstructed from spectrally processed projection data acquired by a spectral imaging apparatus (IA) whilst contrast agent is present in a field of view of the spectral imaging apparatus during acquisition;
segmenting the volume image for contrast agent contributions to obtain a segmented volume image;
forward-projecting the segmented volume image onto the spectrally processed projection data;
establishing a quality indicator data indicative of a change of a concentration of the contrast agent in the field of view during the acquisition, based on a mismatch between the forward-projected segmented volume image, and the spectrally processed projection data, and
providing output data including the quality indicator data.

In yet another aspect there is provided a computer program element, which, when being executed by at least one processing unit, is adapted to cause the processing unit to perform the method.

In another aspect there is provided at least one computer readable medium having stored thereon the program element.

It has been found that if CA concentration varies during projection data acquisition, the 2D CA maps derived from the projection data deliver inconsistent CA information from different viewing angles. These inconsistencies lead to artifacts in the reconstructed 3D CA maps, i.e., to locally wrong quantitative CA concentrations. Thus, the diagnostic value of the 3D CA maps is considerably reduced. The same holds consequently for the VNC images.

Thus, the proposed systema and method allows quantifying the quality of reconstructed imagery in spectral imaging. The quality indicator data being output in particular represents to what extent such reconstructed imagery is corrupted or disturbed, if at all, by artifacts caused by contrast agent concentration changes that happened during acquisition of the (spectral) contrasted projection images.

Thus, the system SYS allows assessing whether the timing of projection data acquisition was with an acceptable error of margins. Specifically, proposed system SYS may allow assessing to what extent the time period of acquisition was outside the plateau/saturation phase PP. Forward projection is used to quantify this quality by determining a mismatch between forward-projected reconstructed spectral imagery, e.g. 3D CA maps, and the original spectral projection images forming the basis for the reconstructed imagery, e.g. 2D CA maps. Corresponding quality indicator data may be output enabling a range of different image assistance tasks and/or control operations which include re-running the acquisition, reconstructing using updated or forward projected data instead of the original projection data and/or using a correction algorithm in the reconstruction as desired.

The proposed system and method are preferably used in interventional imaging such as in trauma departments or Cath Labs where there is need for highly responsive results, delivered in real-time by computationally fast and efficient methods. The proposed method delivers on this front as unwieldy, complex reconstruction or correction schemes can be avoided in some cases when they are not necessary (no artifacts are found), thanks to the proposed quality check measure that can be provided at low computational cost, and in an efficient manner that saves memory and CPU time. Expensive computational and memory costs are only incurred when needed, that is, when quality checker finds the reconstructed spectral volume is indeed inflicted by artifacts caused by in-acquisition variation of contrast agent concentration in the FOV.

*"user"* relates to a person, such as medical personnel or other, operating the imaging apparatus or overseeing the imaging procedure. In other words, the user is in general not the patient.

In terms of *"(tomographic) reconstruction",* a reference herein to a reconstructed *"volume"* in image domain includes a reference to a specific slice.

### BRIEF DESCRIPTION OF THE DRAWINGS

Exemplary embodiments of the invention will now be described with reference to the following drawings, which, unless stated otherwise, are not to scale, wherein:
Fig. 1 shows a schematic block diagram of a contrast agent assisted medical imaging arrangement including a spectral imaging apparatus;
Fig. 2 shows a computer-implemented system of facilitating spectral imaging; and
Fig. 3 is a block diagram of a computer-implemented method of facilitating spectral imaging.

### DETAILED DESCRIPTION OF EMBODIMENTS

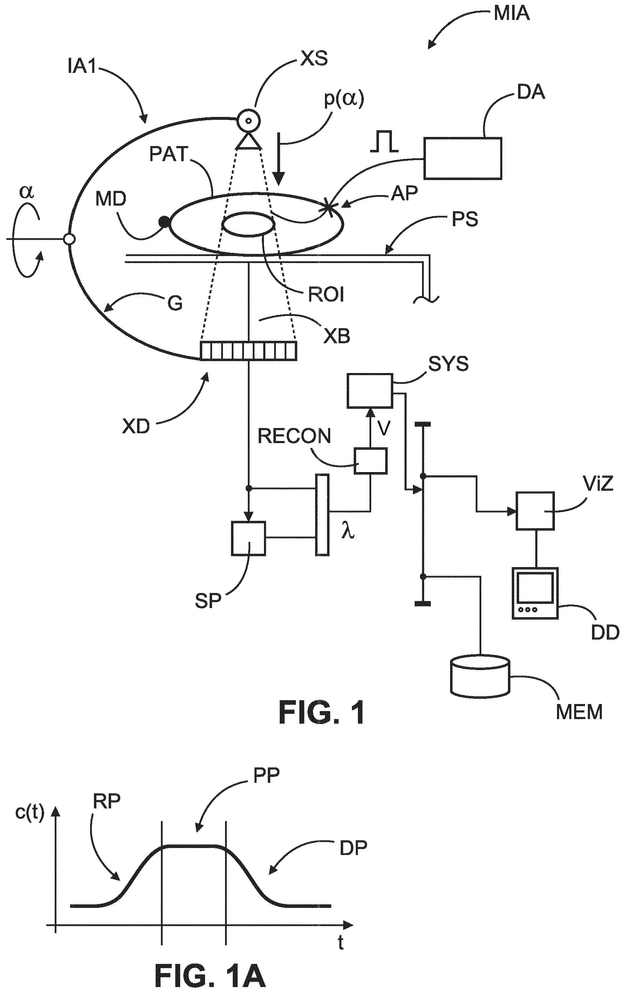

Reference is first made to the block diagram of Fig. 1 which shows in a schematic fashion a medical imaging arrangement MIA. It comprises an imaging apparatus IA ("imager"), preferably X-ray based and of the tomographic type, and a computing system SYS. The imager IA is preferably configured for multi-energy imaging, also known as spectral imaging. The Soft issue imaging is also envisaged

The medical imaging arrangement MIA is operative to produce medical imagery in respect of a patient PAT. The medical arrangement is preferably configured for contrast agent assisted imaging in order to boost contrast for low-attenuating anatomical structures, such as soft tissue of interest in, for example, cardiac or lung imaging. The medical imagery V can be stored, viewed or otherwise processed. The medical imagery may be use by medical used for diagnosis, therapy, planning etc.

Broadly, the imager acquires projection data, and this is then processed by the computing system SYS into the said medical imagery V. The computing system SYS is broadly configured herein to help avoid certain image artifacts that may be incurred due to the use of contrast agent. Such image artifacts are undesirable as they may render the imagery useless as crucial details may be lost. Whilst X-ray based imaging is the main focus herein, other modalities that may be run on contrast agent assisted protocols are not excluded herein such as nuclear imaging (S)PE(C)T, or magnetic resonance imaging (MRI).

Before turning to the computing system SYS in more detail, operation of spectral imager IA in connection with use in a contrast agent assisted protocol is explained first with continued reference to Fig. 1.

The preferably X-ray based imager allows obtaining sectional, preferably 3D, imagery of the ROI. To this end, the imager IA is configured for multi-directional (indicated schematically in the Fig as "•") projection data acquisition around the ROI. The acquisition (path), or "scan", may not necessarily define a complete 360° angular range, but 180°, or even less may be sufficient herein. The acquisition path may not necessarily define a circular arc, although in some most case this may be so as other geometries are also envisaged. Helical paths as are also envisaged herein. Thus, the present disclosure herein is not confined to axial paths, but such are not excluded herein such as in "step-and-shoot" setups.

The imaging apparatus IA may be of the rotational type in some embodiments to effect the multi-directional projection data acquisition operation. Such a rotational imaging apparatus allows collection of projection imagery • along different projection directions P(•) in a scan around the region of interest, such as around the mentioned stricture in a coronary vessel. The imaging apparatus IA includes an X-ray source XS and an X-ray sensitive detector XD. In some embodiments, the X-ray source XS, and opposite thereto, the X-ray sensitive detector XD, are arranged preferably on a rotatable gantry G. The gantry G may have a recess in which an examination region is defined where the patient, or in particular the region of interest, resides during imaging. The rotatable gantry may thus have a "C", "U" or similar shape, such as in C-arm imaging apparatus envisaged herein. A bi-planar imaging setup with two detectors with intersecting imaging axes (for example at right angles) is not excluded herein. Instead of using a C-arm/U-arm system, a CT scanner is also envisaged in some embodiments, of any generation, but is preferably of at least 3^{rd} generation, where X-ray source XS and detector XD are mounted so that they rotate together around the patient. However, for multi-directional projection data acquisition, yet higher generation CT setups are also envisaged, where there is not necessarily mechanical rotation of source and detector. Instead, a stationary ring of detectors around the ROI is used and/or stationary multiple X-sources arranged in a ring around ROI. Cone-beam imaging or other divergent beam geometries are also envisaged herein, and so is imaging with a helical scan path caused by circular path of the X-ray source for example, with translational motion relative to imaging apparatus IA and ROI along patient's longitudinal axis Z. In most cases, this translational motion is caused by a patient bed, with patient on it, being translated during projection data acquisition. However, scan paths with geometry other than circular/helical are not excluded herein.

In addition, whilst digital-at-source imaging, such as flat panel detectors is preferred, where the imagery is natively acquired as digital data, such as setup is not necessarily required. More traditional, cassette-operable, analog imaging methods (based on X-ray films) are not excluded herein. The analog imagery may be digitized post-acquisition for example, by using a digital camera. Imaging based on X-ray image intensifiers, etc with post-digitization is also envisaged in some embodiments.

Generally, imaging/acquisition includes energizing the X-ray source XS, so that an X-ray beam XB issues forth from a focal spot of the source XS, traverses the examination region (with the region of interest in it) and interacts with patient tissue matter. Interaction of X-ray beam XB with tissue matter causes the beam to be modified. The modified beam may then be detected at the detector XD. Conversion circuitry (not shown) converts detected intensities into digital imagery, in particular into projection imagery. The imagery may be used by user in the medical procedure. The patient may reside on a patient support PS such as a table during imaging. Soft tissue such as the one making up the coronary vessels, provide usually poor contrast in native X-ray imagery.

In order to boost contrast, a contrast agent (sometimes "CA" or short), such as an iodine solution, gadolinium, or other, is delivered through an access point AP into the blood flow of the patient prior to or during imaging. The contrast agent may be delivered via a CA delivery apparatus DA, such as a motorized pump, but manual delivery is not excluded herein. The delivery apparatus DA may be operable to deliver a defined volume of contrast agent to the patient. The volume of contrast agent so delivered travels with the blood flow. After a certain period of time contrast agent accumulates at the region of interest such as in or around the stricture. Image acquisition is preferably synchronized with arrival of contrast agent at the ROI. Preferably "contrasted" imagery is acquired whilst there is contrast agent present at the ROI. Optionally, additional "non-contrasted" imagery is acquired whilst no contrast agent is present at the ROI.

The in particular contrasted projection imagery may be acquired in a time series with varying concentration of the contrast agent accumulating at the region of interest. Image acquisition may start before, may continue throughout, and may terminate after accumulation of the contrast agent at the ROI. In general, contrast agent concentration increases over time after delivery, will plateau at a certain maximum value, and will then wash out or decrease over time until completely vanished. Whilst a single or certain discrete number of still images may be acquired in some instances, acquisition of a stream images, or frames, acquired at a suitable frame rate is preferred as this allows real time dynamic observation. Specifically, the acquired stream of projection imagery may be visualized by a visualizer VIZ as a live video feed on display device DD. Alternatively or in addition, still imagery is so visualized. Operation of the proposed system SYS is based on processing such still or stream imagery. The imagery may be stored in an image memory MEM or may be processed otherwise.

X-ray imaging is generally based on the attenuation coefficient of the matter(s) that makes up the region of interest. The attenuation (coefficient) is dependent on the energy of the X-ray beam. In order to harness this energy dependency, a spectral imager IA may be used, configured for spectral imaging. This may include detector-sided or source-sided solutions. Detector-sided solutions include photon-counting detector hardware or a dual-energy setup with dual-layer hardware for example. More than two detector layers may be used although this is less common. Source-side solutions include multi-source setups, kVp switching, filtration and other.

The imaging set-up in spectral imaging allows acquisition of multi-dimensional image data. Such multi-dimensional image data includes projection imagery at different energy levels, instead of acquiring energy integrated imagery as provided by a conventional (non-spectral) X-ray imaging set-up, and along different projection directions •.

Such a spectral CT imaging setup includes a spectral image processor SP. The spectral image processor SP may implement a spectral image processing algorithm, such as material decomposition or other. A range of such spectral image processing algorithms are envisaged herein. The spectral processor SP processes the multi-energy projection imagery • acquired by the source- or detector-sided imaging set-up just described, and computes therefrom spectral imagery that differs from the original input multi-energy projection imagery. Spectral projection imagery is thus computationally derived from the input multi-energy projection imagery.

Such spectral imagery, which may be in projection domain or image domain, may include in particular a contrast-only-image ("VC") as mainly envisaged herein. Such a VC image represents an approximation of an image that may have been obtained if there had been only contrast agent present in the field of view of the imager IA, at the exclusion of other materials. Thus, image contrast in VC is exclusively or pre-dominantly contrast agent focused.

A tomographic reconstructor RECON processes projection data into tomographic/volumetric imagery *R(•*)=*V* as will be described in more detail below. The volumetric data V is spectral image data due to the spectral processor SP. The spectral processor SP may operate in projection domain on projection data • as acquired, or may operate in image domain after reconstruction. For simplicity, the notation "*V*" or "*R(*•)" may be used herein to indicate spectral volumetric imagery in image domain, irrespective of the domain in which the spectral processing SP took place. It is such spectral volumetric data V that is processed by the proposed system SYS. The spectral processor SP may be integrated into the reconstructor RECON.

The spectral processor SP and the proposed system SYS may be implemented on different computing systems, possibly geographically separated such as in a distributed "cloud" architecture. However, implementation on a single computing system is also envisaged herein. Imaging as envisaged herein is preferably attenuation-based, but this is not at the exclusion of other X-ray modalities, such as dark-field imaging and/or phase contrast imaging.

The reconstructor RECON is configured to reconstruct the volumetric imagery V= *R(•)* from the acquired projection data •, the said projection data • acquired whilst at least some contrast agent is present in the field of view of the imager IA, and thus in region of interest including at least a part of a vasculature of interest.

Projection domain is the space situated at the detector where the projection data is detected. Tomographic reconstruction is implemented by reconstructor RECON is a transformation operation *R* from which the volumetric data is computed. The reconstruction transformation operation *R* is a transformation from said projection domain to image domain. It is the said image domain to which the volumetric data *V* is spatially associated. The image domain is the portion of space defined between the X-ray source XD and the detector D, in which space the region of interest (through which the contrast agent passes) resides during imaging. Thus, the region of interest includes in particular the portion of the vasculature VS to be imaged, with, at times, contrast agent residing therein. Conceptually, the image domain may be thought to be made up of a 3D grid of spatial points ("voxels").

Reconstruction operation includes computing image values at each of the grid points (voxels), thus obtaining the volumetric data *R(•)*. Manifold image of reconstruction algorithms are envisaged herein, including FBP, algebraic or iterative or others still. The reconstruction may be iterative. It assigns values to image domain iteratively during various cycles of iteration, based on projection imagery, and may be based on optimization of a functional. During iterations, the reconstruction converges to solution in image domain, that, to the final reconstrued volume V. The functional may measure the consistency of various reconstructed volumes with the measured data. The functional may also include additional functional terms that steer the convergent behavior of the iterative algorithm.

Turning now to the computing system SYS in more detail, this is configured herein for quality control in contrast agent-assisted imaging protocols, harnessing the material specific imaging capabilities of spectral imaging. The system SYS may be configured to alert user to certain image artifacts that may emerge or have emerged in reconstructed volume image, in particular in such contrast agent-assisted imaging applications. The system SYS may facilitate remediating, reducing or avoiding such artifacts.

Upon administration of a certain volume of contrast agent CA, this travels with blood flow to accumulate at the region of interest ("ROI"), for example in a section of a blood vessel of interest, such in the coronaries of patient's heart. The distribution of the liquid blood and contrast agent mix conforms with the vessel's internal structure, in particular its lumen. Spectral imaging allows mapping out the concentration distribution of the contrast agent and thus allows good and targeted image contrast of the anatomy of interest, such as the blood vessel. This in turn allows more precisely informing therapy or diagnosis.

Specifically, concentration distribution of contrast agent over time *t* may be represented by concentration curve *c(t)* as schematically shown in Fig. 1A. At a certain ROI, after passage of a certain arrival time period post administration, in a ramp-up phase RP, contrast agent concentration is expected to increase, followed by a plateau or saturation phase PP where contrast agent concentration achieves a maximum and stays relatively constant for a certain period of time (the "plateau period"), until it drops off again in a drop-off phase DP due to wash out. Contrast agent curve *c(t)* is a function of the location of the region of interest, so behavior/shape of *c(t)* may vary from location to location.

Timing of projection data acquisition is a consideration in contrast assisted imaging protocols as used herein. Ideally, imager IA should be controlled so that projection data acquisition commences at the right time. Specifically, projection data should be acquired during the plateau phase PP, during which contrast agent concentration remains relatively stable due to saturation. Due to various clinical factors (stress level, patient compliance, user fatigue or experience, etc) it may not always be possible to time projection data acquisition such that it occurs entirely during the plateau phase. In these circumstances, the concentration may change either in the ramp-up phase RP or in the drop-off phase DP during projection data acquisition.

Thus, the acquired contrasted projection imagery may include attenuative contributions from changing contrast agent concentrations. It has been found that reconstructing from such projection imagery with contributions from varying contrast agent concentration will lead to image artifacts in the reconstructed imagery V. This is caused by certain violations of consistency assumptions that underly some types of tomographic reconstruction algorithms. For example, pixels that are acquired within a certain time interval or along different views •,•' can be expected to represent the same contrast agent attenuation. Alas, this may not be so if the concentration has changed in this time interval. The reconstruction algorithm may thus not easily be able to assign unambiguously a value to the corresponding voxel in image domain due to the concentration change recorded in projection domain. Especially iterative reconstruction may behave undesirably under such situations. This ambiguity may result in a convergence to a subpar solution, thus leading to artifacts. It is also filtered back-projection (FBP) based reconstruction methods that may be so impacted.

The computer system SYS is configured herein to facilitate reducing such image artifacts in reconstructed imagery that have been caused by changing contrast agent concentration, or the system SYS may alert the user that such artifacts are likely manifest in the current reconstructed imagery. Such facilitation may include alerting the user that the projection data was likely acquired outside the plateau period, and such that reconstruction artifacts are to be expected. The user may then take suitable action, or the system may suggest such action, and, optionally, implement same automatically, or upon user approval input receivable trough suitable UI interaction, etc. Thus, the system may include a user interface (UI) (such as a G(raphical)UI or other). The (G)UI may be configured to alert user and/or allow user controlling which remedial action to take. Thus, the system SYS may be referred to herein as a (image) artifact reducer or alerter.

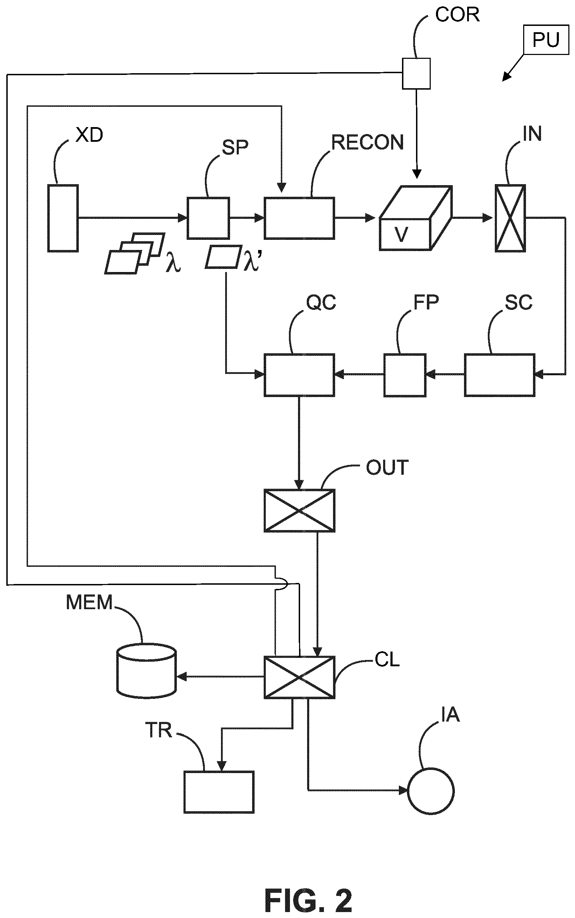

Reference is now made to the block diagram of Fig. 2 which shows components of the artifact alerter system SYS. System SYS may be implemented on a single computing unit PU, or may be implemented in a distributed cloud environment where some or all components are implemented by different computing units/systems/platforms, etc, preferably interconnected in a suitable wired or wireless communication network. The system may be integrated into the imaging apparatus IA, such as in its operating console OC or in an associated work-station, etc. Preferably, system SYS is configured for (quasi-)real-time use on-site, that is, during the imaging session whilst patient is in the examination room, theatre or Cath lab, etc. Thus, system SYS may be implemented in in computing system with high throughput processors, such as those of multi-core design.

Projection data acquired at the detection module XD is processed by a spectral processor SP into spectrally processed projection data. Thanks to the multi-spectral setup, the acquired projection data is multi-energetic data, that is, each pixel in projection domain there is assigned to at least two intensity values, each for a different X-radiation energy range. Spectral processing algorithms such as material decomposition is used to resolve the original multi-energy projection data into spectrally resolved projection data •' where contrast is now material specific, such as for target material of interest. Such target material may be the contrast agent (eg, Iodine, or other). Thus, image contrast in spectral projection data •' may correspond exclusively, or predominately, to the concentration of the contrast agent. A virtual-contrast-agent-only projection image ("VC") may be computed, and such is indeed envisaged herein in embodiments. Thus, each pixel in the VC projection image measures the concentration (mass per volume element) of the specific contrast agent material, which material is known a priori. The so spectrally processed contrast agent only projection data •' (such as VCs) may be understood and referred to herein as "2D contrast agent maps" due to their tailored, CA-specific imaging contrast. There is one or more such 2D contrast agent maps per (main) projection direction (for example, of the central beam).

Various spectral decompositions can be used, such as the 1976 approach by R. E. Alvarez & A. Macovski, published "Energy-selective reconstructions in X-ray computerized tomography", Phys. Med. Biol., vol. 21, No. 5, pp 733, or its cognates. This or similar, related approaches included solving a system of simultaneous linear or non-linear equations based on the attenuation coefficients of different materials at the different energies. The different materials include the target material and at least one other material (or material mix) known/assumed to have been present in the FOV at the time of acquisition.

The so spectrally processed contrast agent only projection data •' (such as VCs) is then passed to the tomographic reconstructor RECON to produce a reconstructed image V in image domain, such as volume V or a single cross-sectional slice. The material specific contrast may, under ideally conditions (no change of contrast agent concentration) carry through into image domain. Thus, the reconstructed volume V may be understood as 3D contrast agent map under ideal condition, which may nevertheless be corrupted by the mentioned artifacts.

The so reconstructed volume V, based on spectrally processed projection data •', is received at input port IN.

A segmentation component SC processes the input volume V to obtain a segmented volume V'.

The segmentor SC may be configured to process the input volume V per voxel. The segmented volume V' may be considered a modified version of the input image V. The segmentation operation may be understood as an image-domain sanity check per voxel. Thus, the segmentation component applies a consistency check at image domain level on whether the reconstructed value per voxel is consistent with the assumption that the respective voxel values represent a realistic contrast agent contribution.

Thus, the segmenter SC may assign a score binary or floating/continuous measure, configured to represent, and to vary with, the extent the voxel indicates a true or realistic contrast agent contribution. If segmenter finds a given voxel is not a true or realistic contrast agent contribution, or is otherwise corrupted, such as deemed unrealistic, the voxel is modified, such as replaced by an estimate, such as default value or is otherwise computed. For example, its value may be interpolated form neighboring voxels that are not found to be corrupted. *"Corruption"* in the present context includes an assumption that value at a given ("*corrupted*") voxel is likely due to contrast agent concentration fluctuations as recorded in pixels in protection domain that are related under projection to that voxel position. The assigning of such sanity score and the voxel modification operation may be two separate processing steps, or may in fact be merged into a single operation.

The consistency check may be based on one of more policies that represent a priori assumptions on the manner in which the voxel values are expected to vary, given that contrast agent was used for respective ROIs. Thus, the policies may be implemented and stored as a respective set of condition, for example in terms of value ranges, topological conditions, etc. Each such policy may be function of contrast agent type and/or ROI. Such policies may be stored in a memory. Thus, different such sanity policies may be used per ROI/application/contrast agent type, etc, each representing certain assumptions on voxel values and their distribution based on prior medical knowledge about the case at hand. A user interface UI may allow user to select which such image-domain sanity check policy the segmenter SC is to apply in order to better comport operation of system SYS to the specifics of the case at hand. For example, UI may be a GUI, arranged to include a tick-box list widget, drop-down widget, or other menu or selection structure-widget.

An image-value threshold or admissible range policy may be used. More specifically, negative values most certainly can be excluded as artifacts as negative concentrations are impossible. Another range-based policy may be used if, for example, small concentration values are not expected in the CA maps (e.g., vessel imaging). The policy may then be configured more aggressively, as even all values below a small positive threshold are then set to zero or other default value. A similar policy can be run, but from the other "end", when for example high concentration values are not expected. The values may thus be capped at a certain predefined max-amount. Second, if for certain reasons, homogeneous CA concentration areas are expected, a topology-based policy may be used to enforce homogeneity and/or connectivity between neighboring voxels, e.g., by processing the image to redistribute values with level set methods, or by a modification of the image driven by a L1-TV regularization, etc.

A forward projector FP is operable to forward-project in a forward-projection operation the so segmented volume V' (an object in image domain) into projection domain and onto the original spectrally processed projection data •' (the 2D contrast agent maps).

The expectation is that if the image-domain based consistency check was done correctly within some error margin, and if there was no contrast agent concentration change recorded by projection data •', then the synthetic projections FP(V') obtained by forward projecting V', should, again within suitable error bounds, correspond (equal) to the spectrally processed input projection data •'. If it does not so correspond, this may be taken as an indication that at least some of the projection data was recorded outside plateau phase / and is/are in fact corrupted.

A quality check module QC implements a quality check measure by establishing quality indicator data indicative of a change of a concentration of the contrast agent in the field of view during the acquisition. For this purpose, for example, the quality checker is configured to compare the forward projected synthesized projections *FP(V')* with the original spectrally processed projection data •' to obtain a trust or mismatch measure (also referred to herein a mismatch information). The quality indicator data may be based on this mismatch measure.

A range of measures may be used by check module QC, such as Euclidian distance, weighted Euclidean distance, or any other measure, taken per pixel. Any distance based on norm *L^{p}, p>0* may be used by quality checker QC. The quality checker module QC may proceed per pixel level in projection domain.

The mismatch measure may be consolidated into a single number, such a binary measure for example that represents whether or not the reconstructed V,V' can be considered faithful reconstruction, that is, whether no contrast agent concentration variations occurred during acquisition of the underlying contrasted projection images. In an example, the volumes V,V' are considered faithful if the projection data was acquired during the plateau phase.

If, on the other hand, there was contrast agent concentration change during acquisition, this will likely manifest in reconstructed image/volume/slice V as image artifacts. It is expected that these artifacts are identifiable by means of determining a mismatch between the forward projected projection imagery and the spectrally processed projection data. A continuous measure, with range in a bounded interval (such as the unit interval [0,1] may be used to quantify the mismatch (if any). A mismatch less than a certain error margin will be considered a match, the conclusion being that there is no corruption, the reconstrued volumes V,V' thus deemed faithful.

Much like in the segmentation by the segmentation module, a thresholding policy may be used by the quality check module QC. The quality check output indicative of the mismatch between the forward-projected volume FP(V') and the spectrally processed projection data • ' may also be provided instead as a map in projection domain indicating the mismatch per pixel location in the projection domain. This can be used to ascertain where in projection domain the mismatch is more prevalent.

The mismatch, and thus the faithfulness of the reconstructed image, may thus be represented as a map in a localized manner. The faithfulness map can be overlaid over one or more of the frames in the projection data •', and can be displayed by visualizer VIZ on the display device DD. Alternatively, or in addition, the mismatch maps may be back-projected and fused with the reconstructed volume V or V', in order to highlight in 3D (in image domain) where the inconsistency is most prevalent. The reconstructed volumes V,V' with the back-projected mismatch map may be displayed by the visualizer VIZ on the display device DD for visual inspection by user. The mismatch values may be thresholded, optionally color coded, to quickly inform user about the spatial distribution of mismatch, and how its severity varies spatially.

Thus, the mismatch may be represented globally by a single scalar, or locally as 2D or 3D maps, depending on the domain in which it is to be used (displayed, processed, etc), in projection domain or image domain.

On occasion, if desired, the mismatch value (a scalar) or the 2D/3D mismatch map may be displayed on their own, rather than in conjunction with the projection data or the reconstructed volume V,V'. Instead of, or in addition to displaying, the mismatch data (scalar or 2D/3D map) may be stored in memory or may otherwise be used, such as for control purposes. Thus, the mismatch data may be used by control interface CL to facilitate a number of useful control operations to assist the user in operating the imaging apparatus IA or other, such as for downstream applications/task post-imaging, or any other task, such as remedial processing, issuing alerts in various form, etc.

For example, transducer TR may be used to convert the mismatch information in a user consumable signal such as a flashlight, auditory alert signal or a haptic signal. In the latter case, UI operational elements (joystick, etc) of imager IA are set into vibration corresponding to the amount of mismatch detected.

If the spectral volume is found to be inflicted by such artifacts as discussed above, the control operation(s) may include any one more of: triggering a re-acquisition of projection data, adjusting of imager settings (kV, mA's etc), rerunning a reconstruction by reconstructor RECON, preferably using a different reconstruction algorithm, controlling a corrector COR to correct the spectral volume V' using an image processing artifact reducing algorithm, scheduling a follow up imaging or other medical session, etc. Any one of such control operations can be used in in any combination/sub-combination with one or more others, or each on their own, as required.

It should be understood that the operations of quality checker QC and/or segmenter SC may be based on a machine learning ("ML") approach. In this embodiment, a respective machine learning model, such as a neural network (preferably of the convolutional type) is used, trained based on training data. The training data may be obtained from medical image data databases. A supervised approach may be used wherein the training data is labelled by a medical expert per pixel/voxel or per patch. The medical expert may assign a score label to what extent the pixel represents a contrast agent contribution or which level of mismatch is a real mismatch or is small enough to pass as a match, etc. Once suitably trained, the segmentation or the quality check can be obtained by applying the trained model to the currently reconstructed volume V', or the mismatch data FP(V') vs •'. In addition to, or instead of machine learning model, fluid dynamics analytical models based on physiological insight may be used by segmentor SC and/or quality checker QC.

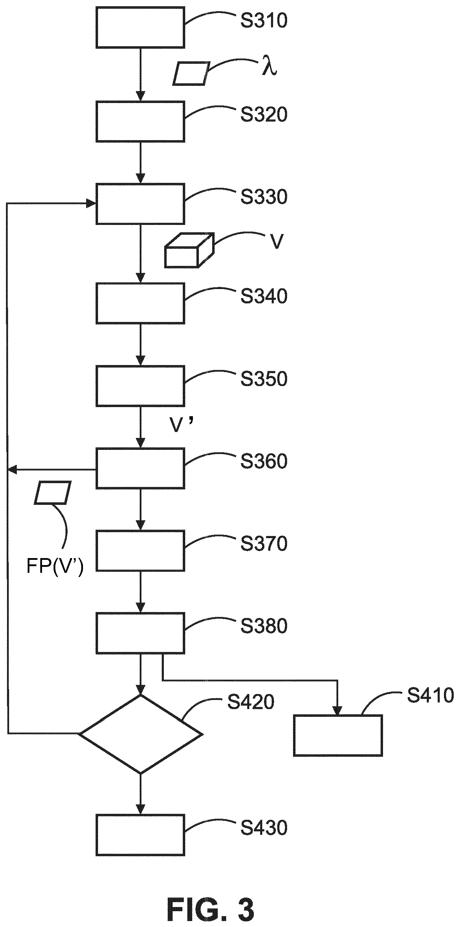

Reference is now made to Fig. 3 which shows a flow-chart of a computer-implemented method of facilitating contrast-agent-assisted tomographic imaging.

At step S310 multi-energy projection data is acquired by an imaging apparatus configured for spectral imaging. As explained above, detector- or source-sided implementations are envisaged herein. The projection data is possibly corrupted as it was acquired at least partly outside plateau phase PP (see Fig 1A above).

At step S320 the acquired projection data is processed by a spectral processing algorithm, such as material decomposition algorithm. In particular, the spectrally processed projection data so obtained represents predominately, or exclusively, contrast agent originating contrast. Any other contributions from other materials or from any other intervening structures are reduced or excluded. Thus a *"virtual contrast agent only"* (VC) projection imagery is obtained. It will be understood, that if there was corruption in •, this corruption feeds through into the spectrally processed projection data • '.

At step S330 the so spectrally processed projection data •' is reconstructed into input spectral volume V. The material specific contrast is carried over into the reconstructed and hence spectral input volume V. The input volume V is received at step S340.

At step S350 the so received reconstructed volume V is then segmented. Segmentation is to be construed broadly and includes assigning at voxel level a respective quantification as to what extent the respective voxel represents true contribution from the contrast agent. If a voxel is not deemed to be so representative, it is replaced, based on a sanity check policy. The result of this operation is a modified/segmented volume V'. The segmentation may provide a segmentation map associated with volume V'. Patch-wise processing instead of, or in combination with, voxel-wise processing is also envisaged and in some cases it may not be necessary to have each and very voxel segmented. On occasion, some regions can be excluded from consideration.

The segmented volume V' is forward-projected at step S360 to obtain a set of synthesized projection data *FP (V').*

This set of synthesized projections *FP (V')* is then compared at step S370 with the original spectrally processed projection data •' to obtain mis-match data as a quality check. If there is no corruption of the projection data (and hence in •') a match is expected. If there is a mismatch, the conclusion is that there is such corruption, and this fact is flagged up. The mis-match data is preferably configured to vary with and represent the amount/severity of the mismatch, and preferably also its spatial distribution in image domain (after back-projection) or in projection domain.

The mismatch data obtained in the comparing at step S370 may be computed by a suitable distance or similarity measure. The mismatch data may be provided as a global scalar value (even, in binary form: "yes mismatch"/"no mismatch"), or may be provided in pixelwise fashion as a 2D map indicative of the amount of mismatch between the two sets of projection data, •' and FP(V'). The mismatch 2D map in projection domain may be back-projected into image domain to provide a 3D mismatch map.

The mismatch information in whichever form is provided at step S380 as output for all manners of further processing, as required.

The mis-match data may be configured to quantify the overall amount of contrast agent variation, e.g., by summing up the detected absolute mismatch over some or all 2D contrast agent maps.

The mismatch data • is an indication for the extent to which the projection imagery • was acquired at least partly during the plateau or whether it was acquired at least partly during the drop-off or ramp-up phase, outside plateau phase, where contrast agent contributions changed. Thus, the mismatched data is a measure for the faithfulness of the reconstruction.

If this measure indicates that the faithfulness is low at step S420, any one of a number of different remedial processing or other steps may be initiated. For example, alternatively, or in addition to step S420, at step S430 an alert signal of the auditory, haptic or visual type may be issued, once mismatch exceeds a certain pre-defined threshold.

As to the remedial processing step S420, this may include running a second reconstruction operation. This may be based on the synthesized projection data FP(V'), instead of, or in addition to, the original spectrally processed set •'. For example, a computationally more-demanding reconstruction algorithm may be used for the second reconstruction that is designed to take into account contrast agent fluctuations in the underlying projection data.

Thus, the proposed system and method allow saving valuable CPU time as such computationally more taxing reconstruction algorithms are only run when needed. The computationally more-demanding reconstruction algorithm may be run also on the original data • or •'. Similarly, and as an alternative, in the remedial processing step S420, a more involved image corrector algorithm may be run by corrector COR that processes the reconstructed spectral volume V', in case artifacts are found by quality check step to provide a corrected spectral volume. Such correction algorithm may also be computationally expensive, as it attempts to remove artifacts caused by contrast agent concentration changes as recorded by the underlying projection data •,•'. Thus, thanks to the prosed quality checking step S370, this computational cost is incurred only when needed (when artifacts as found to be present).

In addition or instead, in the remedial processing step S420, the imager IA may be controlled so as to acquire a new set of projection data with newly administered contrast agent, where a repeat attempt is made this time to capture the projection data within the plateau period. However, this will expose the patient to a higher dose of contrast agent and radiation, which in normal circumstances is to be avoided because of the negative health implications. Thus, acquiring new set of contrasted projections is likely to be done only in exceptional circumstances, such as extreme corruption. Then again, the proposed method helps avoid such retakes, and the associated efficiency costs and health costs: retakes are done only when needed.

At step S410 the faithfulness data or mismatch data is displayed. This may be graphically rendered, and/or may be displayed numerically. In one embodiment, image portions to which the mismatch pertains are localized, either in projection domain or in image domain.

A graphical indication may be provided such as by color- or grey level coding in respect of the mis-match data, in order to indicate where the mismatch originated from, which portions are more affected, and to the degree they are, etc. Thus, it may be that not the whole of the volume is unfaithful, but only certain portions thereof. Thus, "localization" as used herein is the operation of mapping the mismatch data into 2D (projection domain) or 3D (image domain), in order to represent the distribution of faithfulness.

The mis-match data provides user with a quality/reliability measure feedback for the acquisition. User may then choose to repeat the acquisition. Alternatively, retake is triggered automatically. Similarly, user may decide if an improved reconstruction (with a more advanced, concentration fluctuation compensating reconstruction) should be performed, or such is triggered automatically. In both cases, the automatic triggering is through a suitable control interface CL.

It will be understood that any of the components of system SYS (Fig. 2) and any of the processing steps (Fig. 3) described above in terms of per voxel/pixel level, may be carried out instead per patches (subsets of voxels), an optional consideration if saving of computing time is of interest.

The components of system SYS may be implemented as one or more software modules, run on one or more general-purpose processing units PU such as a workstation associated with the imager IA, or on a server computer associated with a group of imagers.

Alternatively, some or all components of system SYS may be arranged in hardware such as a suitably programmed microcontroller or microprocessor, such an FPGA (field-programmable-gate-array) or as a hardwired IC chip, an application specific integrated circuitry (ASIC), integrated into the imaging system SYS. In a further embodiment still, the system SYS may be implemented in both, partly in software and partly in hardware.

The different components of system SYS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is defined by the appended claims.

## Claims

1. System (SYS) for image processing, comprising:
an input interface (IN) for receiving, when the system is in use, a volume image reconstructed from spectrally processed projection data acquired by a spectral imaging apparatus (IA) whilst contrast agent is present in a field of view of the spectral imaging apparatus during acquisition;
a segmentor (SC) component configured to segment the volume image for contrast agent contributions to obtain a segmented volume image;
a forward-projector (FP) configured to forward-project the segmented volume image onto the spectrally processed projection data;
a quality checker (QC) configured to establish quality indicator data indicative of a change of a concentration of the contrast agent in the field of view during the acquisition, based on a mismatch between the forward-projected segmented volume image, and the spectrally processed projection data, and
an output interface (OUT) for providing output data including the quality indicator data.

2. System of claim 1, comprising a visualizer (VIZ) configured to generate, based on the output data, a graphics display for display on a display device (DD), wherein in the graphics display the mismatch, if any, is localized in at least part of the projection data or in the volume image.

3. System of any one of the previous claims, comprising a control interface (CL) configured to request, based on the output data, any one or more of: i) issuing an alert signal, ii) additional processing in relation to the projection data, iii) an additional acquisition of new projection data.

4. System of claim 3, wherein the alert signal is indicative whether such additional processing or additional acquisition is warranted, or that such additional processing or additional acquisition is recommended, and wherein the additional processing or additional acquisition is requested based on user input.

5. System of claim 3 or 4, wherein the alert signal includes any one or more of: i) an audio alert signal, ii) a haptic feedback-signal, iii) a visual alert signal.

6. System of any one of claims 3-5, wherein the additional processing includes any one or more of i) an additional reconstruction operation by a reconstructor (RECON) to reconstruct a second volume image based on the new projection data or based on the forward projections obtained by forward projector (FP), ii) a correcting by a corrector (COR) of the volume image for the mismatch, based on the said output data.

7. System of claim 1, wherein the spectrally processed projection data comprises 2D contrast agent (2D CA) maps representing the concentration of the contrast agent, and the volume image comprises a 3D contrast agent (3D CA) map.

8. System of claim 1, wherein the segmentor (SC) is configured to apply a consistency check on the volume image at image domain level, the consistency check indicating whether a reconstructed value per voxel is consistent with an assumption that the respective voxel values represent a realistic contrast agent contribution.

9. System of claim 8, wherein the consistency check includes an image-value threshold policy and/or a topology-based policy enforcing homogeneity and/or , connectivity between neighboring voxels.

10. An imaging arrangement (MIA) including the system of any one of the previous claims, and further including one or more of: i) the display device (DD), ii) the imaging apparatus.

11. Computer-implemented of image processing, comprising:
receiving (S340) a volume image reconstructed from spectrally processed projection data acquired by a spectral imaging apparatus (IA) whilst contrast agent is present in a field of view of the spectral imaging apparatus during acquisition;
segmenting (S350) the volume image for contrast agent contributions to obtain a segmented volume image;
forward-projecting (S360) the segmented volume image onto the spectrally processed projection data;
establishing (S370) a quality indicator data indicative of a change of a concentration of the contrast agent in the field of view during the acquisition, based on a mismatch between the forward-projected segmented volume image, and the spectrally processed projection data, and
providing (S380) output data including the quality indicator data.

12. A computer program element, which, when being executed by at least one processing unit, is adapted to cause the processing unit to perform the method as per claim 11.

13. At least one computer readable medium having stored thereon the program element of claim 12.

## Patentansprüche

1. System (SYS) zur Bildverarbeitung, umfassend:
eine Eingabeschnittstelle (IN) zum Empfangen eines Volumenbildes, wenn das System in Verwendung ist, das aus spektral verarbeiteten Projektionsdaten wiederhergestellt wird, die von einer Spektralbildgebungseinrichtung (IA) erfasst werden, während beim Erfassen ein Kontrastmittel in einem Sichtfeld der Spektralbildgebungseinrichtung vorhanden ist;
eine Segmentierer (SC), der konfiguriert ist, um das Volumenbild für Kontrastmittelbeiträge zu segmentieren, um ein segmentiertes Volumenbild zu erhalten;
einen Vorwärtsprojektor (FP), der konfiguriert ist, um das segmentierte Volumenbild auf die spektral verarbeiteten Projektionsdaten vorwärts zu projizieren;
einen Qualitätsprüfer (QC), der konfiguriert ist, um Qualitätsindikatordaten zu ermitteln, die auf eine Änderung einer Konzentration des Kontrastmittels im Sichtfeld bei der Erfassung hinweisen, basierend auf einer Nichtübereinstimmung zwischen dem vorwärts projizierten segmentierten Volumenbild und den spektral verarbeiteten Projektionsdaten, und
eine Ausgabeschnittstelle (OUT) zum Bereitstellen von Ausgabedaten, die die Qualitätsindikatordaten beinhalten.

2. System nach Anspruch 1, das einen Visualisierer (VIZ) umfasst, der konfiguriert ist, um basierend auf den Ausgabedaten eine Grafikanzeige zur Anzeige auf einer Anzeigevorrichtung (DD) zu erzeugen, wobei in der Grafikanzeige die Nichtübereinstimmung, falls vorhanden, in mindestens einem Teil der Projektionsdaten oder im Volumenbild lokalisiert wird.

3. System nach einem der vorstehenden Ansprüche, umfassend eine Steuerschnittstelle (CL), die konfiguriert ist, um basierend auf den Ausgabedaten eines oder mehrere anzufordern von: i) Ausgeben eines Warnsignals, ii) zusätzlichem Verarbeiten in Bezug auf die Projektionsdaten, iii) einem zusätzlichen Erfassen neuer Projektionsdaten.

4. System nach Anspruch 3, wobei das Warnsignal darauf hinweist, ob eine solche zusätzliche Verarbeitung oder zusätzliche Erfassung gerechtfertigt ist, oder dass eine solche zusätzliche Verarbeitung oder zusätzliche Erfassung empfohlen wird, und wobei die zusätzliche Verarbeitung oder zusätzliche Erfassung basierend auf Benutzereingabe angefordert wird.

5. System nach Anspruch 3 oder 4, wobei das Warnsignal eines oder mehrere beinhaltet von: i) einem akustischen Warnsignal, ii) einem haptischen Feedback-Signal, iii) einem visuellen Warnsignal.

6. System nach einem der Ansprüche 3-5, wobei die zusätzliche Verarbeitung eines oder mehrere beinhaltet von: i) einem zusätzlichen Wiederherstellungsvorgang durch einen Wiederhersteller (RECON), um ein zweites Volumenbild basierend auf den neuen Projektionsdaten oder basierend auf den vom Vorwärtsprojektor (FP) erhaltenen Vorwärtsprojektionen wiederherzustellen, und ii) Korrigieren des Volumenbildes hinsichtlich der Nichtübereinstimmung durch einen Korrektor (COR), basierend auf den Ausgabedaten.

7. System nach Anspruch 1, wobei die spektral verarbeiteten Projektionsdaten 2D-Kontrastmittelkarten (2D-CA) umfassen, die die Konzentration des Kontrastmittels darstellen, und das Volumenbild eine 3D-Kontrastmittelkarte (3D-CA) umfasst.

8. System nach Anspruch 1, wobei der Segmentierer (SC) konfiguriert ist, um auf Bilddomänenebene eine Konsistenzprüfung auf das Volumenbild anzuwenden, wobei die Konsistenzprüfung darauf hinweist, ob ein wiederhergestellter Wert pro Voxel mit der Annahme konsistent ist, dass die jeweiligen Voxelwerte einen realistischen Kontrastmittelbeitrag darstellen.

9. System nach Anspruch 8, wobei die Konsistenzprüfung eine Bildwertschwellenrichtlinie und/oder eine topologiebasierte Richtlinie zur Durchsetzung von Homogenität und/oder Konnektivität zwischen benachbarten Voxeln beinhaltet.

10. Bildgebungsanordnung (MIA), die das System nach einem der vorstehenden Ansprüche beinhaltet, und weiter eines oder mehrere beinhaltet von: i) der Anzeigevorrichtung (DD), ii) der Bildgebungseinrichtung.

11. Computerimplementierte Bildverarbeitung, umfassend:
Empfangen (S340) eines Volumenbildes, wenn das System in Verwendung ist, das aus spektral verarbeiteten Projektionsdaten wiederhergestellt wird, die von einer Spektralbildgebungseinrichtung (IA) erfasst werden, während beim Erfassen ein Kontrastmittel in einem Sichtfeld der Spektralbildgebungseinrichtung vorhanden ist;
Segmentieren (S350) des Volumenbildes für Kontrastmittelbeiträge, um ein segmentiertes Volumenbild zu erhalten;
Vorwärtsprojizieren (S360) des segmentierten Volumenbildes auf die spektral verarbeiteten Projektionsdaten;
Ermitteln (S370) von Qualitätsindikatordaten, die auf eine Änderung einer Konzentration des Kontrastmittels im Sichtfeld bei der Erfassung hinweisen, basierend auf einer Nichtübereinstimmung zwischen dem vorwärts projizierten segmentierten Volumenbild und den spektral verarbeiteten Projektionsdaten, und Bereitstellen (S380) von Ausgabedaten, die die Qualitätsindikatordaten beinhalten.

12. Computerprogrammelement, das, wenn es von mindestens einer Verarbeitungseinheit ausgeführt wird, dazu angepasst ist, die Verarbeitungseinheit zu veranlassen, das Verfahren nach Anspruch 11 durchzuführen.

13. Mindestens ein computerlesbares Medium, das darauf gespeichert das Programmelement nach Anspruch 12 aufweist.

## Revendications

1. Système (SYS) de traitement d'image, comprenant :
une interface d'entrée (IN) pour recevoir, lorsque le système est en cours d'utilisation, une image de volume reconstruite à partir de données de projection traitées spectralement acquises par un appareil d'imagerie spectrale (IA) alors qu'un agent de contraste est présent dans un champ de vision de l'appareil d'imagerie spectrale pendant l'acquisition ;
un composant segmenteur (SC) configuré pour segmenter l'image de volume pour des contributions d'agent de contraste afin d'obtenir une image de volume segmentée ;
un projecteur vers l'avant (FP) configuré pour projeter vers l'avant l'image de volume segmentée sur les données de projection traitées spectralement;
un dispositif de commande de qualité (QC) configuré pour établir des données d'indicateur de qualité indiquant un changement de concentration de l'agent de contraste dans le champ de vision pendant l'acquisition, sur la base d'une discordance entre l'image de volume segmentée projetée vers l'avant et les données de projection traitées spectralement, et
une interface de sortie (OUT) pour fournir des données de sortie incluant les données d'indicateur de qualité.

2. Système selon la revendication 1, comprenant un visualiseur (VIZ) configuré pour générer, sur la base des données de sortie, un affichage graphique destiné à être affiché sur un dispositif d'affichage (DD), dans lequel, dans l'affichage graphique, la discordance, le cas échéant, est localisée dans au moins une partie des données de projection ou dans l'image de volume.

3. Système selon l'une quelconque des revendications précédentes, comprenant une interface de commande (CL) configurée pour demander, sur la base des données de sortie, un ou plusieurs parmi : i) l'émission d'un signal d'alerte, ii) un traitement supplémentaire par rapport aux données de projection, iii) une acquisition supplémentaire de nouvelles données de projection.

4. Système selon la revendication 3, dans lequel le signal d'alerte indique si un tel traitement supplémentaire ou une telle acquisition supplémentaire est justifié, ou si un tel traitement supplémentaire ou une telle acquisition supplémentaire est recommandé, et dans lequel le traitement supplémentaire ou l'acquisition supplémentaire est demandé sur la base d'une entrée utilisateur.

5. Système selon la revendication 3 ou 4, dans lequel le signal d'alerte inclut un ou plusieurs parmi : i) un signal d'alerte audio, ii) un signal de rétroaction haptique, iii) un signal d'alerte visuel.

6. Système selon l'une quelconque des revendications 3-5, dans lequel le traitement supplémentaire inclut une ou plusieurs parmi : i) une opération de reconstruction supplémentaire par un reconstructeur (RECON) pour reconstruire une seconde image de volume sur la base des nouvelles données de projection ou sur la base des projections vers l'avant obtenues par le projecteur vers l'avant (FP), ii) une correction par un correcteur (COR) de l'image de volume pour la discordance, sur la base desdites données de sortie.

7. Système selon la revendication 1, dans lequel les données de projection traitées spectralement comprennent des cartes d'agent de contraste 2D (2D CA) représentant la concentration de l'agent de contraste, et l'image de volume comprend une carte d'agent de contraste 3D (3D CA).

8. Système selon la revendication 1, dans lequel le segmenteur (SC) est configuré pour appliquer une vérification de cohérence sur l'image de volume au niveau du domaine d'image, la vérification de cohérence indiquant si une valeur reconstruite par voxel est cohérente avec une hypothèse selon laquelle les valeurs de voxel respectives représentent une contribution d'agent de contraste réaliste.

9. Système selon la revendication 8, dans lequel la vérification de cohérence inclut une politique de seuil de valeur d'image et/ou une politique basée sur la topologie imposant l'homogénéité et/ou une connectivité entre voxels voisins.

10. Dispositif d'imagerie (MIA) incluant le système selon l'une quelconque des revendications précédentes, et incluant en outre un ou plusieurs parmi : i) le dispositif d'affichage (DD), ii) l'appareil d'imagerie.

11. Procédé de traitement d'image mis en œuvre par ordinateur, comprenant :
la réception (S340) d'une image de volume reconstruite à partir de données de projection traitées spectralement acquises par un appareil d'imagerie spectrale (IA) alors qu'un agent de contraste est présent dans un champ de vision de l'appareil d'imagerie spectrale pendant l'acquisition ;
la segmentation (S350) de l'image de volume pour des contributions d'agent de contraste afin d'obtenir une image de volume segmentée ;
la projection vers l'avant (S360) de l'image de volume segmentée sur les données de projection traitées spectralement ;
l'établissement (S370) de données d'indicateur de qualité indiquant un changement d'une concentration de l'agent de contraste dans le champ de vision pendant l'acquisition, sur la base d'une discordance entre l'image de volume segmentée projetée vers l'avant et les données de projection traitées spectralement, et la fourniture (S380) de données de sortie incluant les données d'indicateur de qualité.

12. Élément de programme informatique qui, lorsqu'il est exécuté par au moins une unité de traitement, est adapté pour amener l'unité de traitement à réaliser le procédé selon la revendication 11.

13. Au moins un support lisible par ordinateur sur lequel est stocké l'élément de programme selon la revendication 12.
